# EUROPEAN PATENT SPECIFICATION

(11) **EP 4 277 584 B1**
(45) Date of publication and mention of the grant of the patent: **25.03.2026**
(21) Application number: 22739250.3
(22) Date of filing: 02.01.2022
(51) Int. Cl.: A61F 13/42, G01N 33/50, G01N 33/52, A61B 10/00, A61B 5/00

(54) **WEARABLE DEVICE FOR DETECTION OF AMNIOTIC FLUID**
AM KÖRPER TRAGBARE VORRICHTUNG ZUR ERKENNUNG EINER FRUCHTWASSER
DISPOSITIF PORTABLE POUR LA DÉTECTION DE LIQUIDE AMNIOTIQUE

(30) Priority: 12.01.2021 US 202163136272 P
(43) Date of publication of application: 22.11.2023
(73) Proprietor: Savyon Diagnostics Ltd, 7761003 Ashdod (IL)
(72) Inventor: GAL, Ido, 4438106 Kfar Saba (IL); ZUCKER, Rita, 3486101 Haifa (IL); SLEMAN, Nour, Nazareth (IL); YAKOV, Erez, 8469809 Be'er Sheva (IL); SOLVATZOV, Tanya, 3061626 Or Akiva (IL)
(74) Representative: AWA Denmark A/S
(86) International application number: PCT/IL2022/050002
(87) International publication number: WO 2022/153291

(56) References cited:
- WO-A1-2014/026188
- CN-A- 105 277 656
- CN-A- 107 576 653
- CN-A- 107 576 653
- JP-A- H05 123 324
- US-A- 6 126 597
- US-A- 6 126 597
- US-A1- 2018 142 414
- US-A1- 2019 240 373
- US-A1- 2020 197 231

## Description

### BACKGROUND OF THE INVENTION

Premature rupture of the amniotic sac is a common disorder that happens to occur in around 10% of pregnant women. Failure to treat premature rapture may cause intra-amnion infection, disruption in fetal lung development, and even perinatal death.

Several approaches for detecting amniotic leakage are known in the art, including chromatographic detection of a color change in a diagnostic article following contact with vaginal secretion.

US 2019/240373 A1 discloses an article for detecting amniotic fluid according to the preamble of claim 1.

There is an unmet need for an improved diagnostic system that can differentiate between amniotic fluid and an interfering biological fluid, such as, urine, with minimal false positive results, while providing a reliable indication which is stable over time, and while reducing the amount of time required to get the reliable result.

### SUMMARY OF THE INVENTION

The invention relates to an article according to claim 1.

### BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.
FIG. 1 is a schematic description of a secretion monitoring article (1 - membrane layer, 2 - transfer layer, 3 - absorbent layer, 4 - base layer).
FIG. 2 is a photograph of pantyliner article containing airlaid absorbent layers of varying densities after exposure to urine simulants at different pH levels.
FIG. 3 is a photograph of pantyliner article containing airlaid absorbent layers of varying densities after exposure to amniotic fluid.

### DETAILED DESCRIPTION OF THE INVENTION

A description of example embodiments of the invention follows.

The invention provides an article comprising an indicator composition. Furthermore, non-claimed methods of use thereof for obtaining an immediate, clear and reliable identification of amniotic fluid leakage are disclosed, the methods being useful for understanding the invention.

Advantageously, articles described herein may operate right after contacting the vaginal secretion. Specifically, the articles and diagnostic compositions disclosed herein do not require any drying time and/or do not require waiting for urine evaporation. The detection of amniotic fluid (or other fluids) by the articles disclosed herein is a one-step process, namely, upon contact of a liquid with an article comprising the diagnostic composition, detection is carried out on the article of the invention, right after being in contact with the fluids. Furthermore, the article of the invention differentiates between amniotic fluid and an interfering biological fluid, such as urine, with minimal false positive results. Without wishing to be bound by any theory or mechanism, it is assumed that this advantage is rendered by the unique combination of the absorbent layer present in the article and the ingredients in the diagnostic formulations of the invention, such as an aliphatic carboxylic acid, a hydrophilic surfactant, a non-cyclic plasticizer, an ion-balance reagent, an indicator agent or any combination thereof.

There is provided an article for detecting amniotic fluid, comprising the features of claim 1.

Furthermore, the articles of the invention enable initial self-detection and do not require the patient to visit health-care professional in order to distinguish between positive and false-positives. Accordingly, using the article of the invention saves a significant amount of time required to determine if amniotic fluid is actually leaking.

The terms "amniotic fluid leakage" and "premature rupture of the amniotic sac" are interchangeable and relate to the spontaneous rupture of the membranes (the amniotic sac is often termed "fetal membrane") 24 or more hours before the onset of labor. About 30-50% of premature ruptures occur before the 37^{th} week of pregnancy. As amniotic fluid leakage is associated with a significant increase in the risk of an intrauterine infection and disturbance of development of the fetal lung system, definitive and reliable diagnosis of the rupture is crucial. Intrauterine penetration of such infections increases both maternal and perinatal morbidity and mortality in about ten percent of the cases. Immediate diagnosis of a rupture at 38 to 40 weeks of pregnancy is crucial, since once detected delivery should be induced as soon as possible. The rupture diagnosis is also important before 37 weeks of pregnancy because it enables prevention of intra-amnion infection and the stimulation of fetal lung development.

Due to the severe consequences of amniotic fluid leakage, pregnant women undergo severe stress and often go to a health-care professional upon secretion of any liquid from the vicinity of the vagina. The health-care professional looks for the presence of amniotic fluid by checking the pH of the vaginal secretions - amniotic fluid is having a pH of between 6.7 and 7.5. Since pregnant women often have urinary incontinence and since urine typically has a pH of between 5.0 and 8.0, if only pH is checked, a false positive result may occur: urine being identified as amniotic fluid. Consequently, it is necessary that a vaginal secretion be examined using a microscope for the presence of a fern-shaped pattern indicative of amniotic fluid.

As the time between the fluid secretion and the arrival at the health-care professional may be long, there is often no evidence of amniotic fluid upon examination. The secretion may mistakenly assumed to be urine, which may result with tragic consequences. On the other hand, the healthcare professional may decide to err on the side of caution, misdiagnosing the secretion of urine as amniotic fluid leading to an unnecessary hospitalization and stress.

Various diagnostic indicators are known, however, an obstacle of many is that they often provide false positives due to changes in pH on drying, interfering biological fluids and repetitive cycles of drying/wetting. The false positive readings can be stressful and time consuming to the user. The article of the invention isaccurate, reliable, and convenient for the user compared to those known in prior art.

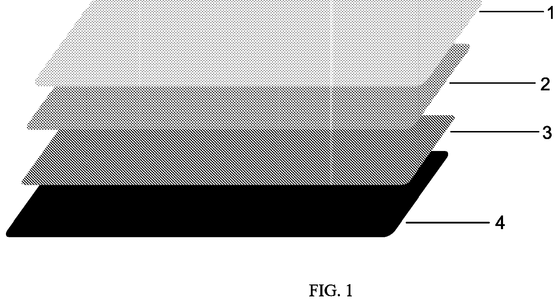

In an embodiment of the invention, there is provided an article for detecting amniotic fluid, comprising: a membrane layer **1;** a transfer layer **2** having a first side and a second side; an absorbent layer **3** having a first side and a second side; and a base layer **4,** wherein the membrane layer is adjacent to the first side of the transfer layer; the second side of the transfer layer is adjacent to the first side of the absorbent layer; and the second side of the absorbent layer is adjacent to the base layer, and further wherein the transfer layer comprises an indicator composition disposed thereon comprising: one or more indicator composition polymers; a non-cyclic plasticizer; a hydrophilic surfactant; an C₁₋₁₆ aliphatic carboxylic acid; an ion-balance reagent; and an indicator agent (FIG. 1).

The liquid first interacts with the membrane layer of the article. The membrane layer can comprise a hydrophilic coating, which helps the liquid permeate through the membrane layer. The hydrophilic coating can comprise a cationic coating, such as Silastol PH 26, manufactured by Schill+Seilacher. The liquid then comes in contact with the transfer layer, which comprises the indicator composition. The transfer layer and the membrane layer can each independently comprise a woven or nonwoven polyolefin, such as nonwoven polypropylene. In some embodiments, the density of the transfer and the membrane layer can be from 6 grams per square meter (GSM) to 40 GSM, such as, from 8 GSM to 30 GSM, 8 GSM to 20 GSM, 8 GSM to 16 GSM or from 12 GSM to 14 GSM.
As used herein, the term "nonwoven" refers to a material made from staple fiber (short) and long fibers (continuous long), bonded together by chemical, mechanical, heat or solvent treatment. Nonwoven materials (e.g., fabrics) are broadly defined as sheet or web structures bonded together by entangling fiber or filaments (and by perforating films) mechanically, thermally or chemically. They are flat or tufted porous sheets that are made directly from separate fibers, molten plastic or plastic film. They are not made by weaving or knitting and do not require converting the fibers to yarn.

As used herein, the term "woven" refers to a material in (e.g., fabric), in which two distinct sets of yarns or threads are interlaced at right angles to form the material.

As used herein, the term "polyolefin" refers to a polymer that can be produced via polymerization of an alkene (olefin).

After the liquid permeates the transfer layer, it contacts the absorbent layer. The absorbent layer can comprise an airlaid layer, which assists in wicking liquid through the transfer layer. An airlaid layer comprises an airlaid material.

As used herein, "airlaid material" refers to a nonwoven material comprising one or more layer comprising natural pulp fibers, or a mixtures of natural fluff pulp and binding fibers, that form a homogeneous and continuous web. "Natural fluff pulp", as used herein, refers to a lignocellulosic fibrous material prepared by chemically separating cellulose fibers from long fiber softwoods.

The airlaid layer can also comprise a superabsorbent polymer (SAP). As used herein, the term "superabsorbent polymer" refers to a polymer that can absorb up to 300 times its weight of water or aqueous solutions. A SAP can comprise a synthetic polymer, such as sodium polyacrylate, a polyacrylamide copolymer, an ethylene maleic anhydride copolymer, a cross-linked carboxymethylcellulose, a polyvinyl alcohol copolymer, or a cross-linked polyethylene oxide, or a natural or a semisynthetic polymers, such as a polysaccharide or a starch grafted copolymer. The SAP in the airlaid layer absorbs the aqueous liquid that permeates the absorbent layer.

The airlaid layers of the article of the invention can have varying densities (also referred to as airlaid weight). For example, the density of the airlaid layer can be from 100 grams per square meter (GSM) to 750 GSM, such as from 250 GSM to 500 GSM.

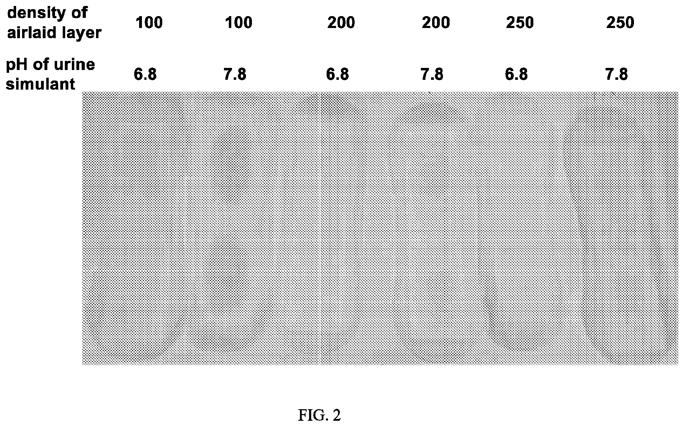

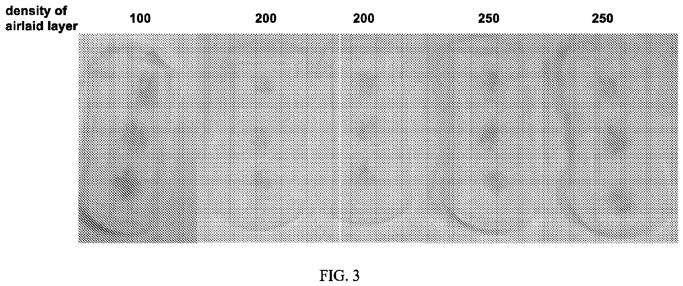

The photographs shown in FIGs. 2 and 3 demonstrate that the airlaid layer density affects the ability of the panty liner article to differentiate between a urine simulant and amniotic fluid. FIG. 2 shows photographs of three sets of two panty liners each with varying densities of the airlaid layer: 100 GSM, 200 GSM, and 250 GSM. The panty liners also contained a nonwoven polypropylene membrane layer with Silastol PHP 26 coating and a nonwoven polypropylene transfer layer coated with the following indicator composition: 43.8 wt.% cellulose acetate, 17.5 wt.% cellulose acetate butyrate, 25.0 wt% triethyl citrate, 6.6 wt.% poloxamer 407, 5.5 wt.% citric acid, 1.2 wt.% tridodecylmethyl ammonium chloride, and 0.53 wt.% nitrazine yellow.

One of the panty liners in each set was treated with a urine simulant with pH 6.8, and the other one was treated with a urine simulant with pH 7.8. Presence of a blue stain on the panty liner indicated a false positive result, since the panty liner should not produce a color change in the presence of urine. As the images in FIG. 2 demonstrate, while the urine simulant with pH 6.8 provided no false positive results in all the three airlaid layers of various densities, the urine simulant with pH 7.8 gave a strong false positive reading in the panty liner with 100 GSM airlaid layer, and a fainter one in the panty liner with 150 GSM airlaid layer.

Meanwhile, the photographs in FIG. 3 show that similar sets of panty liners with 100 GSM, 200 GSM, and 250 GSM airlaid layers provided equally strong positive signal when exposed to real amniotic fluid regardless of the density of the airlaid layer in the panty liner.

The data in FIGs. 2 and 3 demonstrate that the density of the airlaid layer affects selectivity of the panty liner. In an article with a higher GSM airlaid layer urine (or urine stimulant) is absorbed more efficiently, thus shortening the time during which the urine can interact with the indicator composition in the transfer layer and produce a false positive signal. Meanwhile, amniotic fluid contains proteins and solid particles that are not present in urine and as a result amniotic fluid pass through the membrane layer and the transfer layer more slowly to be absorbed by the airlaid layer, thus allowing it to stay in contact with the indicator composition in the transfer layer for a time sufficient to produce a positive signal.

In some embodiments, the article further comprises a first adhesive layer between the membrane layer and the transfer layer, and a second adhesive layer between the transfer layer and the absorbent layer. In some embodiments, the first adhesive layer and/or the second adhesive layer comprise a hot melt adhesive material.

In some embodiments, the base layer is designed to face the inner surface, i.e., the crotch portion of an undergarment. The base layer may permit a passage of air or vapor out of the absorbent layer, while still blocking the passage of liquids. Any liquid-impermeable material may generally be utilized to form the base layer. For example, one suitable material that may be utilized is a microporous polymeric film, such as polyethylene or polypropylene. In particular embodiments, a polyethylene film is utilized that has a thickness in the range of about 0.2 millimeters to about 5.0 millimeters, and particularly between about 0.5 to about 3.0 millimeters.

In some embodiments, each of the one or more indicator composition polymers are cellulose esters comprising partially acetylated cellulose polymeric chains, including, but not limited to, cellulose acetate in which substantially each one of positions 2, 3 and 6 of the monomeric glucose units of the cellulose polymer, consists of either free hydroxyl or acetate. In some embodiments, the polymers are cellulose esters comprising substantially acetylated cellulose polymeric chains, including, but not limited to, cellulose triacetate. In some embodiments, the polymers are cellulose esters comprising partially esterified cellulose polymeric chains, wherein the esterification comprises partial acetylation and partial butyrilation, including, but not limited to, cellulose acetate butyrate, in which substantially each one of positions 2, 3 and 6 of the monomeric glucose units of the cellulose polymer consists of either free hydroxyl, acetate or butyrate. In some embodiments, the cellulose is a combination of cellulose acetate and cellulose acetate butyrate. In some embodiments, the ratio therebetween cellulose acetate and cellulose acetate butyrate is between 2-3. In some embodiments, the ratio therebetween cellulose acetate and cellulose acetate butyrate is between 2.2-2.8. In some embodiments, the ratio therebetween cellulose acetate and cellulose acetate butyrate is about 2.5. In some embodiments, the ratio therebetween cellulose acetate and cellulose acetate butyrate is between 2.4-2.6. In some embodiments, the polymers are cellulose esters comprising partially esterified cellulose polymeric chains, wherein the esterification comprises partial acetylation and partial propionilation, including, but not limited to, cellulose acetate propionate, in which substantially each one of positions 2, 3 and 6 of the monomeric glucose units of the cellulose polymer consists of either free hydroxyl, acetate or propionate.

As used herein, "non-cyclic plasticizer" refers to a plasticizer that does not contain a cyclic moiety, e.g., a cycloalkyl, a cycloalkenyl, a heterocyclyl, and aryl, in its structure. In some embodiments, the non-ionic plasticizer is a low molecular weight plasticizer. Typically, a low molecular weight plasticizer is a plasticizer having a molecular weight of less than 600 g/mol. In some embodiments, the plasticizer is having a molecular weight of less than 500 g/mol. In some embodiments, the plasticizer is having a molecular weight of less than 400 g/mol. In some embodiments, the plasticizer is having a molecular weight of less than 300 g/mol.

In a non-claimed example useful for understanding the invention, low molecular weight plasticizers include C₁₋₁₆ alkyl , such as, tributyl phosphate, trioctyl phosphate, tricresyl phosphate, and triphenyl phosphate; C₁₋₁₆ alkyl citrate and citrate esters, such as trimethyl citrate, triethyl citrate, tributyl citrate, acetyl triethyl citrate, and trihexyl citrate; C₁₋₁₆ dialkyladipates such as dioctyladipate (DOA; also referred to as bis(2-ethylhexyl)adipate), diethyl adipate, di(2-methylethyl)adipate, and dihexyladipate; C₁₋₁₆ dialkyl tartrates such as diethyl tartrate and dibutyl tartrate; C₁₋₁₆ dialkylsebacates such as diethyl sebacate, dipropylsebacate and dinonylsebacate; C₁₋₁₆ dialkyl succinates such as diethyl succinate and dibutyl succinate; C₁-₁₆ alkyl glycolates, C₁-₁₆ alkyl glycerolates, glycol esters and glycerol esters such as glycerol diacetate, glycerol triacetate (triacetin), glycerol monolactatediacetate, methyl phthalyl ethyl glycolate, butyl phthalyl butyl glycolate, ethylene glycol diacetate, ethylene glycol dibutyrate, triethylene glycol diacetate, triethylene glycol dibutyrate and triethylene glycol dipropionate; acetylated monoglycerides and mixtures thereof.

In some non-claimed examples useful for understanding the invention, the non-cyclic plasticizer comprises low molecular weight plasticizers, including, but not limited to, C₁₋₁₆ alkyl citrate and citrate esters, such as, trimethyl citrate, triethyl citrate, tributyl citrate, acetyl triethyl citrate, and trihexyl citrate, acetylated monoglycerides and mixtures thereof.

In some non-claimed examples useful for understanding the invention, the non-cyclic plasticizer is a tri-ester plasticizer. In some embodiments, the low molecular weight plasticizers comprise a triester structure. Such plasticizers include, but are not limited to, C₁₋₁₆ alkyl citrate and citrate esters, such as trimethyl citrate, triethyl citrate, tributyl citrate, acetyl triethyl citrate and trihexyl citrate. In some embodiments, the plasticizer is triethyl citrate. In some embodiments, the plasticizer is 1,2-cyclohexane dicarboxylic acid diisononyl ester.

In some non-claimed examples useful for understanding the invention, the non-cyclic plasticizer comprises triethyl citrate.

As used herein, the term "hydrophilic surfactant" refers to a surfactant that has Hydrophilic Lipophilic Balance (HLB) value over 10. HLB is equal to the molecular weight percent of the hydrophilic portion of the surfactant divided by 5. For example, HLB number of Poloxamer 407 is 22, therefore Poloxamer 407 is a hydrophilic surfactant (Dumortier et al., "A Review of Poloxamer 407 Pharmaceutical and Pharmacological Characteristics," 2006, Pharm. Res. 23(12):2709-2328), In some embodiments, the hydrophilic surfactant is a polymer. In some embodiments, the surfactant comprises a block copolymer surfactant. In some embodiments, the polymeric surfactant comprises a tri-block copolymer surfactant. In some embodiments, the polymeric surfactant comprises poly(ethylene oxide) and/or copolymers thereof. In some embodiments, the polymeric surfactant comprises poly(propylene oxide) and/or copolymers thereof. In some embodiments, the block copolymer surfactant comprises copolymer of poly(ethylene oxide) and poly(propylene oxide) and/or copolymers thereof.

In some embodiments, the hydrophilic surfactant is a non-ionic surfactant. In some embodiments, the non-ionic surfactant comprise a non-ionic polymeric surfactant. In some embodiments, the surfactant is selected from the group of poloxamer, alkyl poly(ethylene oxide), copolymers of poly(ethylene oxide) and poly(propylene oxide), octylglucoside, decylmaltoside, cetyl alcohol, oleyl alcohol, cocamidemonoethanolamine (cocamide MEA), cocamidediethanolamine (cocamide DEA), cocamidetriethanolamine (cocamide TEA), polysorbate, such as, Tween^{®} (e.g. Tween 20^{®} or Tween 80^{®}), lecithin and combinations thereof.

In some embodiments, the non-ionic polymeric surfactant is selected from the group of poly(ethylene oxide) and copolymers of poly(ethylene oxide), poly(propylene oxide), polysorbate, such as, Tween^{®} (e.g. Tween 20^{®} or Tween 80^{®}) and combinations thereof.

In some embodiments, the surfactant comprises poloxamer, polysorbate, and a combination thereof.

In some embodiments, the surfactant comprises a poloxamer. In some embodiments, the surfactant comprises poloxamer 407.

In some embodiments, the surfactant comprises a polysorbate. In some embodiments, the non-ionic surfactant is other than ionic surfactants, such as, aliquot 336 (Tricaprylylmethylammonium chloride).

The term "poloxamer" as used herein refers to nonionic triblock copolymers comprising a central hydrophobic chain of polyoxypropylene (poly(propylene oxide)) and two hydrophilic chains of polyoxyethylene (poly(ethylene oxide)). Poloxamers are commonly termed "P" followed by three digits, the first two times 100 refer to the estimated molecular mass of the polyoxypropylene core, and the last digit times 10 is the percentage polyoxyethylene content. For example, P407 refers to a poloxamer with a polyoxypropylene molecular mass of 4,000 g/mol and 70% polyoxyethylene content.

The term "polysorbate" as used herein generally refers to polyoxyethylene derivative of sorbitan monolaurate. Common commercial brand names for polysorbates include Tween^{®}, such as, Tween 20^{®} or Tween 80^{®}. The number following the polyoxyethylene part refers to the total number of oxyethylene groups found in the molecule. The number following the polysorbate part is related to the type of fatty acid associated with the polyoxyethylene sorbitan part of the molecule. Monolaurate is indicated by 20, monopalmitate is indicated by 40, monostearate by 60 and monooleate by 80.

Without wishing to be bound by any theory or mechanism, the non-ionic polymeric surfactant distributes within the urine and as a result retention time of urine is reduced thereby avoiding false positive results from urine.

In some embodiments, the article of the invention contains a C₁-₁₆ aliphatic carboxylic acid as a competitive reagent. Without being bound by any theory or mechanism, the C₁₋₁₆ aliphatic carboxylic acid creates a complex with the ammonium ions in urine. This interaction results in reduction of interference from urine, including urine with pH level of 7.0 units, and even higher, and therefore improves indicator specificity.

In some embodiments, the C1-16 aliphatic carboxylic acid is selected from citric acid, oxalic acid, tartaric acid, succinic acid, glutaric acid, lactic acid, pyruvic acid, hydroxypropionic acid, hydroxyvaleric acid, adipic acid, and suberic acid, or a combination thereof.

In some embodiments, the C1-16 aliphatic carboxylic acid is citric acid.

Comparison of articles useful for understanding the invention containing citric acid and salicylic acid as competitive reagents demonstrates that presence of citric acid provides an article with an ability to avoid false positive results due to the presence of urine over a wider range of pH values. Articles A and B with identical membrane layer, transfer layer, and absorbent layer were prepared. The transfer layers in the two articles contained the following indicator compositions:

| **Indicator composition component** | **Material** | **Article A, wt.% of material** | **Article B, wt.% of material** |
|---|---|---|---|
| indicator composition polymer | cellulose acetate | 43.8 | 43.3 |
| indicator composition polymer | cellulose acetate butyrate | 17.5 | 17.3 |
| ion balance reagent | TDMAC | 1.2 | 1.1 |
| non-cyclic plasticizer | triethyl citrate | 25.0 | 24.7 |
| competitive reagent | salicylic acid | 0.0 | 6.5 |
| competitive reagent | citric acid | 5.5 | 0.0 |
| hydrophilic surfactant | poloxamer 407 | 6.6 | 6.5 |
| indicator agent | nitrazine yellow | 0.5 | 0.5 |

Both panty liners were exposed to urine simulants at different pH values. The results are shown in Table 1.

**Table 1. Exposure of articles A and B to urine simulant at various pH values.**

| **pH** | **6** | **6.5** | **7** | **7.5** | **8** |
|---|---|---|---|---|---|
| Article A | Neg* | Neg | Neg | Pos | Pos |
| Article B | Pos** | Pos | Pos | Pos | Pos |

| | | | | | |
|---|---|---|---|---|---|
| *Negative (weakly colored stain or no stain); ** Positive (strongly colored stain) | | | | | |

The data in Table 1 demonstrate that at pH levels of the urine stimulant below 7.5 article A, which contained citric acid, provided a negative signal, thus correctly detecting absence of the amniotic fluid. Meanwhile, article B, which contained salicylic acid, provided false positive results in the full range of the examine pH values.

In some embodiments, the ion-balance reagent is selected from the group consisting of: di(C₆₋₂₀ alkyl)dimethyl ammonium chloride, N-methyl-N,N-bis(C₆₋₂₀ alkanoyloxyethyl)-N-(2-hydroxyethyl) ammonium methylsulfate, vinylbenzyldimethylcocoammonium chloride, methyl trioctyl ammonium chloride tricaprylylmethyl ammonium chloride, tridodecylmethyl ammonium chloride and cetyltimethyl ammonium chloride. Each possibility represents as separate embodiment of the invention.

Any suitable ion-balance reagent may be used, including, tridodecylmethyl ammonium chloride (TDMAC; CAS 7173-54-8) or cetyltrimethyl ammonium chloride (CTAC; CAS 112-02-7). In some embodiments, the ion balance reagent is a combination of ion-balance reagents. In some embodiments, the ion-balance reagent is TDMAC.

In some embodiments, the indicator agent is selected from the group of chemical compounds having negatively charged functional groups. In some embodiments, the indicator agent is a weak acid. In some embodiments, the indicator agent is selected from the group consisting of nitrazine yellow, thymol blue, bromthymol blue, xylenol blue, bromoxylenol blue, phenol red, m-cresol purple, chlorophenol red, bromcresol purple, alizarin, neutral red, and cresol red. A list of other suitable indicators can be found, for example, in U.S. Patent No. 5,897,834.

In some embodiments, the indicator agent is selected from the group consisting of: cresol red, alizarin, bromocresol purple, chlorophenol red, nitrazine yellow, bromthymol blue, bromoxylenol blue, neutral red, phenol red, thymol blue, xylenol blue and m-cresol purple. Each possibility represents as separate embodiment of the present invention.

As used herein, the term "about" refers to a ±10% range of the given value (including the range endpoints). For example, a percentage of about 2% refers to the percentage (and including) 1.8%, the percentage (and including) 2.2%, and all percentages in between.

As used herein, "alkyl" refers a saturated aliphatic branched or straight-chain monovalent hydrocarbon radical having the specified number of carbon atoms. An alkyl group can have from 1 to 20 carbon atoms, such as 1 to 16 carbon atoms, or 6 to 20 carbon atoms. "C₁₋₁₆ alkyl" refers to a radical having from 1 to 16 carbon atoms in a linear or branched arrangement. A "lower alkyl" group refers to an alkyl group comprising 1 to 4 carbon atoms. Also included within the definition of "alkyl" are those alkyl groups that are optionally substituted. Suitable subsitutions include, but are not limited to, halogen, OH, CN, alkoxy, amino, haloalkyl, nitro, lower alkylamino and the like.

As used herein, the term "aryl," means a carbocyclic aromatic system containing one or more rings, which may be attached together in a pendent manner or may be fused. In some embodiments, an aryl has one, two or three rings. In one aspect, the aryl has 6 to 18 ring atoms. The term "aryl" encompasses aromatic radicals such as phenyl, naphthyl, tetrahydronaphthyl, indanyl, biphenyl, phenanthryl, anthryl and acenaphthyl. An "aryl" group can have 1 to 4 substituents, such as lower alkyl, hydroxyl, halo, haloalkyl, nitro, cyano, alkoxy, amino, lower alkylamino, and the like.

The term "aliphatic" or "aliphatic group," as used herein, denotes a monovalent hydrocarbon radical that is straight-chain (i.e., unbranched), branched, or cyclic (including fused, bridged, and spiro-fused polycyclic). An aliphatic group can be saturated or can contain one or more units of unsaturation, such as double or triple carbon-carbon bonds, but is not aromatic. An aliphatic group can be optionally substituted as described herein. "C₁₋₁₆ aliphatic" refers to an aliphatic radical having from 1 to 16 carbon atoms. An aliphatic radical does not comprise aromatic or heteroaromatic moieties. Also included within the definition of "aliphatic" are those aliphatic groups that are optionally substituted. Suitable subsitutions include, but are not limited to lower alkyl, hydroxyl, halo, haloalkyl, nitro, cyano, alkoxy, amino, lower alkylamino, and the like.

The term "hydrophilic polymer", "hydrophilic surfactant" or "hydrophilic coating" refer to the amount of water vapor absorbed by the material such as polymer, surfactant, or coating, at 100% relative humidity. Thus, "hydrophobic polymers" typically refer to polymers that absorb only up to 1 wt. % water at 100% relative humidity, while moderately hydrophilic polymers absorb 1-10% wt. % water and hydrophilic polymers are capable of absorbing more than 10 wt. % of water. In some embodiments, the polymers of the invention are water-insoluble polymers. Without wishing to be bound by any theory or mechanism of action, presence of hydrophilic components in the panty liner article improves the penetration of the fluid to be tested into the panty liner.

There is provided a non-claimed method useful for understanding the invention, the method being suitable for diagnosing amniotic fluid leakage in vaginal secretion and comprising:
positioning an article described herein to receive a biological fluid secreted from the subject, contacting said article with vaginal secretion; and viewing the article, wherein a color change indicates the presence of amniotic fluid.

Optionally, said color change is a dark stain relative to background of the secretion monitoring article. In some embodiments, the dark stain is a dark blue stain or a dark green stain.

Optionally, said viewing is performed within less than 15 minutes after said contacting. In some embodiments, said viewing is performed within less than 14 minutes, less than 13 minutes, less than 12 minutes, less than 11 minutes, less than 10 minutes, less than 9 minutes, less than 8 minutes, less than 7 minutes, less than 6 minutes, less than 5 minutes, less than 4 minutes, less than 3 minutes, or less than 2 minutes after said contacting.

In some embodiments, the article further comprises a means for mounting the article to facilitate the collection of the secreted biological fluid. An example of a mounting means includes, but is not limited to, adhesive strips associated with the article. In some embodiments, the article further comprises release tapes, each covering each of the adhesive strips. Covering as used herein also means protecting (e.g. from drying out). Typically, the user removes the release tapes to expose the adhesive strips of the article and places the article in the crotch portion of their undergarment, thereby preventing the article from moving out of position during regular use. Types of adhesive compounds that can be used are well known in the art.

In some embodiments, the article provides a visible indication of amniotic fluid. In some embodiments, the visual indication is stable for about a week.

The terms "stable indication" and "irreversible indication" are interchangeably used herein to describe an indication, typically a color indication, that once obtained remains sufficiently unaltered for the time required for clinical examination by a professional. Preferably, the color change is stable for at least 48 hours, more preferably at least 72 hours, and in some embodiments, preferably the color change is stable for about a week.

In some embodiments, there is provided a kit comprising the article for detecting amniotic fluid in vaginal secretion, and instructions for use of the article in order to detect amniotic fluid in vaginal secretion.

In some embodiments, the kit further comprises a color scale presenting the color expected to appear upon contact of the article with amniotic fluid and the color expected to appear upon contact of the article with urine.

The present disclosure relates to an article for detecting amniotic fluid, comprising: a membrane layer; a transfer layer having a first side and a second side; an absorbent layer having a first side and a second side; and a base layer, wherein: the membrane layer is adjacent to the first side of the transfer layer; the second side of the transfer layer is adjacent to the first side of the absorbent layer; and the second side of the absorbent layer is adjacent to the base layer, and further wherein: the transfer layer comprises an indicator composition disposed thereon comprising: one or more indicator composition polymers a non-cyclic plasticizer; a hydrophilic surfactant; a C₁₋₁₆ aliphatic carboxylic acid; an ion-balance reagent; and an indicator agent.

The membrane layer comprises a woven polyolefin layer or a nonwoven polyolefin layer. In some embodiment, the nonwoven polyolefin layer comprises polypropylene. The membrane layer further comprises a hydrophilic coating disposed thereon. In some embodiments, the hydrophilic coating comprises a cationic surfactant.

In some embodiments, the transfer layer comprises a woven polyolefin layer or a nonwoven polyolefin layer. In some embodiments, nonwoven polyolefin layer comprises polypropylene.

In some embodiments, the absorbent layer comprises an airlaid layer. In some embodiments, the airlaid layer comprises natural fluff pulp. In some embodiments, the airlaid layer further comprises binding fibers. In some embodiments, the density of the airlaid layer is from 200 grams per square meter (GSM) to 750 GSM. In some embodiments, the density of the airlaid layer is from 200 GSM to 350 GSM. For example, the density of the airlaid layer is 200, 250 GSM, 300 GSM, 350 GSM, 400 GSM, 450 GSM, 500 GSM, 550 GSM 600 GSM, 650 GSM, or 700 GSM.

In some embodiments, the airlaid layer further comprises a superabsorbent polymer. In some embodiments, the superabsorbent polymer comprises sodium polyacrylate, a polyacrylamide copolymer, an ethylene maleic anhydride copolymer, a cross-linked carboxymethylcellulose, a polyvinyl alcohol copolymer, a cross-linked polyethylene oxide, a starch grafted copolymer, or a combination thereof.

In some embodiments, each of the one or more indicator composition polymers is selected from cellulose, a cellulose ester, and a copolymer thereof. In some embodiments, each of the one or more indicator composition polymers is a cellulose ester. In some embodiments, each of the one or more indicator composition polymers is selected from cellulose acetate, cellulose acetate butyrate, cellophane, cellulose nitrate, cellulose propionate, cellulose propionate butyrate, cellulose diacetate, cellulose triacetate, cellulose ethers, carboxymethyl cellulose, or any copolymer thereof. In some embodiments, the one or more indicator composition polymers comprise cellulose acetate. In some embodiments, the one or more indicator composition polymers comprise cellulose acetate butyrate. In some embodiments, each of the one or more indicator composition polymers is present in the amount from about 10 wt.% to about 60 wt.% of the indicator composition. For example, each of the one or more indicator composition polymers is present in the amount of about 10 wt.%, about 15 wt.%, about 20 wt.%, about 25 wt.%, about 30 wt.%, about 35 wt.%, about 40 wt.%, about 45 wt.%, about 50 wt.%, about 55 wt.%, or about 60 wt.% of the indicator composition.

In some non-claimed examples useful for understanding the invention, the non-cyclic plasticizer is a di-C₁₋₁₆ alkyl ester of a dicarboxylic acid or a tri-C₁₋₁₆ alkyl ester of a tricarboxylic acid. In some embodiments, the non-cyclic plasticizer has a molecular weight from about 200 g/mol to about 600 g/mol. For example, the non-cyclic plasticizer has a molecular weight from about 250 g/mol to about 550 g/mol, or from about 300 g/mol to about 500 g/mol, such as about 200 g/mol, about 250 g/mol, about 300 g/mol, about 350 g/mol, about 400 g/mol, about 450 g/mol, about 500 g/mol, about 550 g/mol, or about 600 g/mol. Optionally, the non-cyclic plasticizer is triethyl citrate. Optionally, the non-cyclic plasticizer is present in the amount from about 15 wt.% to about 40 wt.% of the indicator composition. For example, the non-cyclic plasticizer is present in the amount of about 15 wt.%, about 20 wt.%, about 25 wt.%, about 30 wt.%, about 35 wt.%, or about 40 wt.%, about 45 wt.% of the indicator composition.

In some embodiments, the hydrophilic surfactant is selected from a poloxamer, a polysorbate, and ethyl-L-lactate. In some embodiments, the hydrophilic surfactant is a poloxamer. In some embodiments, the hydrophilic surfactant is poloxamer 407. In some embodiments, the hydrophilic surfactant is present in the amount from about 2 wt.% to about 10 wt.% of the indicator composition. For example, the hydrophilic surfactant is present in the amount of about 2 wt.%, about 3 wt.%, about 4 wt.%, about 5 wt.%, about 6 wt.%, or about 7 wt.%, about 8 wt.%, about 9 wt.%, or about 10 wt.% of the indicator composition.

The C₁₋₁₆ aliphatic carboxylic acid is selected from citric acid, oxalic acid, tartaric acid, succinic acid, glutaric acid, lactic acid, pyruvic acid, hydroxypropionic acid, hydroxyvaleric acid, adipic acid, and suberic acid, or a combination thereof. In some embodiments, the C₁₋₁₆ aliphatic carboxylic acid is citric acid. In some embodiments, the C₁₋₁₆ aliphatic carboxylic acid is present in the amount from about 3 wt.% to about 8 wt.% of the indicator composition. For example, the C₁₋₁₆ aliphatic carboxylic acid is present in the amount of about 3 wt.%, about 3.5 wt.%, about 4 wt.%, about 4.5 wt.%, about 5 wt.%, about 5.5 wt.%, about 6 wt.%, about 6.5 wt.%, about 7 wt.%, about 7.5 wt.%, or about 8 wt.% of the indicator composition.

In some embodiments, the ion-balance reagent is a quaternary ammonium salt or a tertiary ammonium salt. In some embodiments, the ion-balance reagent is selected from di(C₆₋₂₀ alkyl)dimethyl ammonium chloride, N-methyl-N,N-bis(C₆₋₂₀ alkanoyloxyethyl)-N-(2-hydroxyethyl) ammonium methylsulfate, vinylbenzyldimethylcocoammonium chloride, methyl trioctyl ammonium chloride, tricaprylylmethyl ammonium chloride, tridodecylmethyl ammonium chloride, and cetyltrimethyl ammonium chloride, or a combination thereof. In some embodiments, the ion-balance reagent is tridodecylmethyl ammonium chloride. In some embodiments, the ion-balance reagent is present in the amount from about 0.5 wt.% to about 2 wt.% of the indicator composition. For example, the ion-balance reagent is present in the amount of about 0.5 wt.%, about 0.6 wt.%, about 0.7 wt.%, about 0.8 wt.%, about 0.9 wt.%, about 1.0 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, or about 2.0 wt.% of the indicator composition.

In some embodiments, the indicator agent is negatively charged. In some embodiments, the indicator agent is selected from nitrazine yellow, cresol red, alizarin, bromocresol purple, chlorophenol red, bromothymol blue, bromoxylenol blue, neutral red, phenol red, thymol blue, xylenol blue or m-cresol purple. In some embodiments, the indicator agent is nitrazine yellow. In some embodiments, the indicator agent is present in the amount from about 0.1 wt.% to about 1 wt.% of the indicator composition. For example, the indicator agent is present in the amount of about 0.1 wt.%, about 0.2 wt.%, about 0.3 wt.%, about 0.4 wt.%, 0.5 wt.%, about 0.6 wt.%, about 0.7 wt.%, about 0.8 wt.%, about 0.9 wt.%, or about 1.0 wt.% of the indicator composition.

In some embodiments, the membrane layer comprises a nonwoven polypropylene layer and a hydrophilic coating disposed thereon; the transfer layer comprises a nonwoven polypropylene layer; and the absorbent layer comprises an airlaid layer comprising a superabsorbent polymer, wherein the density of the airlaid layer is from 200 grams GSM to 350 GSM.

In some embodiments, the one or more indicator composition polymers are cellulose acetate and cellulose acetate butyrate; the non-cyclic plasticizer is triethyl citrate; the hydrophilic surfactant is poloxamer 407; the C₁₋₁₆ aliphatic carboxylic acid is citric acid; the ion-balance reagent is tridodecylmethyl ammonium chloride: and the indicator agent is nitrazine yellow.

In some embodiments, the indicator composition comprises 43.8 wt.% cellulose acetate, 17.5 wt.% cellulose acetate butyrate, 25.0 wt% triethyl citrate, 6.6 wt.% poloxamer 407, 5.5 wt.% citric acid, 1.2 wt.% tridodecylmethyl ammonium chloride, and 0.53 wt.% nitrazine yellow.

In some embodiments, the indicator composition consists of cellulose acetate, cellulose acetate butyrate, triethyl citrate, poloxamer 407, citric acid, tridodecylmethyl ammonium chloride, and nitrazine yellow.

In some embodiments, the indicator composition consists of 39-47 wt.% cellulose acetate, 20-15 wt.% cellulose acetate butyrate, 22-28 wt% triethyl citrate, 8-5 wt.% poloxamer 407, 8-5 wt.% citric acid, 1.15-1.27 wt.% tridodecylmethyl ammonium chloride, and 0.48-0.6 wt.% nitrazine yellow.

In some embodiments, the membrane layer comprises a nonwoven polypropylene layer and a hydrophilic coating disposed thereon; the transfer layer comprises a nonwoven polypropylene layer; and the absorbent layer comprises an airlaid layer comprising a superabsorbent polymer, wherein the density of the airlaid layer is from 200 GSM to 350 GSM, and further wherein: the transfer layer comprises an indicator composition disposed thereon, comprising 39-47 wt.% cellulose acetate, 20-15 wt.% cellulose acetate butyrate, 22-28 wt% triethyl citrate, 8-5 wt.% poloxamer 407, 8-5 wt.% citric acid, 1.15-1.27 wt.% tridodecylmethyl ammonium chloride, and 0.48-0.6 wt.% nitrazine yellow. In some embodiments, the membrane layer comprises a nonwoven polypropylene layer and a hydrophilic coating disposed thereon; the transfer layer comprises a nonwoven polypropylene layer; and the absorbent layer comprises an airlaid layer comprising a superabsorbent polymer, wherein the density of the airlaid layer is from 200 GSM to 350 GSM, and further wherein: the transfer layer comprises an indicator composition disposed thereon, comprising 43.8 wt.% cellulose acetate, 17.5 wt.% cellulose acetate butyrate, 25.0 wt% triethyl citrate, 6.6 wt.% poloxamer 407, 5.5 wt.% citric acid, 1.2 wt.% tridodecylmethyl ammonium chloride, and 0.53 wt.% nitrazine yellow.

While this invention has been particularly shown and described with references to example embodiments thereof, it will be understood by those skilled in the art that various changes in form and details may be made therein without departing from the scope of the invention encompassed by the appended claims.

## Claims

1. An article for detecting amniotic fluid, comprising:
a membrane layer (1);
a transfer layer (2) having a first side and a second side;
an absorbent layer (3) having a first side and a second side; and
a base layer (4), wherein:
the membrane layer is adjacent to the first side of the transfer layer;
the second side of the transfer layer is adjacent to the first side of the absorbent layer; and
the second side of the absorbent layer is adjacent to the base layer,
and further wherein:
the transfer layer comprises an indicator composition disposed thereon comprising:
one or more indicator composition polymers;
a non-cyclic plasticizer;
a hydrophilic surfactant;
a C₁₋₁₆ aliphatic carboxylic acid;
an ion-balance reagent; and
an indicator agent;
wherein the membrane layer comprises a woven polyolefin layer or a nonwoven polyolefin layer, and
**characterized in that**
the non-cyclic plasticizer is a C1-16 aliphatic carboxylic acid selected from citric acid, oxalic acid, tartaric acid, succinic acid, glutaric acid, lactic acid, pyruvic acid, hydroxypropionic acid, hydroxyvaleric acid, adipic acid, and suberic acid, or a combination thereof; and **in that**
the membrane layer further comprises a hydrophilic coating disposed thereon.

2. The article of claim 1, wherein the transfer layer comprises a woven polyolefin layer or a nonwoven polyolefin layer.

3. The article of claims 1 and 2, wherein the absorbent layer comprises an airlaid layer which optionally comprises natural fluff pulp and binding fibers.

4. The article of claim 3, wherein the density of the airlaid layer is from 200 grams per square meter (GSM) to 750 GSM optionally from 200 GSM to 350 GSM.

5. The article of any one of Claims 1-4, wherein the one or more indicator composition polymers comprise cellulose acetate and/or cellulose acetate butyrate

6. The article of any one of Claims 1-5, wherein the non-cyclic plasticizer is triethyl citrate.

7. The article of any one of Claims 1-6, wherein the hydrophilic surfactant is poloxamer 407.

8. The article of any one of Claims 1-7, wherein the C₁₋₁₆ aliphatic carboxylic acid is citric acid.

9. The article of any one of Claims 1-8, wherein the ion-balance reagent is tridodecylmethyl ammonium chloride.

10. The article of any one of Claims 1-9, wherein the indicator agent is selected from nitrazine yellow, cresol red, alizarin, bromocresol purple, chlorophenol red, bromothymol blue, bromoxylenol blue, neutral red, phenol red, thymol blue, xylenol blue or m-cresol purple.

11. The article of any one of Claims 1-10, wherein the indicator composition comprises 43.8 wt.% cellulose acetate, 17.5 wt.% cellulose acetate butyrate, 25.0 wt% triethyl citrate, 6.6 wt.% poloxamer 407, 5.5 wt.% citric acid, 1.2 wt.% tridodecylmethyl ammonium chloride, and 0.53 wt.% nitrazine yellow.

12. The article of any one of Claims 1-12, wherein the indicator composition consists of cellulose acetate, cellulose acetate butyrate, triethyl citrate, poloxamer 407, citric acid, tridodecylmethyl ammonium chloride, and nitrazine yellow.

13. The article of Claim 1, wherein:
the membrane layer comprises a nonwoven polypropylene layer;
the transfer layer comprises a nonwoven polypropylene layer; and
the absorbent layer comprises an airlaid layer comprising a superabsorbent polymer, wherein the density of the airlaid layer is from 200 GSM to 350 GSM,
and further wherein:
the indicator composition comprises 39-47 wt.% cellulose acetate, 15-20 wt.% cellulose acetate butyrate, 22-28 wt% triethyl citrate, 5-8 wt.% poloxamer 407, 5-8 wt.% citric acid, 1.15-1.27 wt.% tridodecylmethyl ammonium chloride, and 0.48-0.6 wt.% nitrazine yellow.

## Patentansprüche

1. Artikel zum Nachweis von Fruchtwasser, umfassend:
eine Membranschicht (1);
eine Übertragungsschicht (2) mit einer ersten Seite und einer zweiten Seite;
eine absorbierende Schicht (3) mit einer ersten Seite und einer zweiten Seite; und
eine Basisschicht (4), wobei:
die Membranschicht an die erste Seite der Übertragungsschicht angrenzt;
die zweite Seite der Übertragungsschicht an die erste Seite der absorbierenden Schicht angrenzt; und
die zweite Seite der absorbierende Schicht an die Basisschicht angrenzt,
und wobei weiterhin:
die Übertragungsschicht eine darauf angeordnete Indikatorzusammensetzung umfasst, die Folgendes umfasst:
ein oder mehrere Indikatorzusammensetzungspolymere;
einen nichtcyclischen Weichmacher;
ein hydrophiles Tensid;
eine aliphatische C₁₋₁₆-Carbonsäure;
ein Ionenausgleichsreagenz; und
ein Indikatormittel;
wobei die Membranschicht eine gewebte Polyolefinschicht oder eine Polyolefinvliesschicht umfasst, und
**dadurch gekennzeichnet, dass**
der nichtcyclische Weichmacher eine aliphatische C1-16-Carbonsäure ausgewählt aus Citronensäure, Oxalsäure, Weinsäure, Bernsteinsäure, Glutarsäure, Milchsäure, Brenztraubensäure, Hydroxypropionsäure, Hydroxyvaleriansäure, Adipinsäure und Korksäure oder eine Kombination davon ist; und dadurch, dass
die Membranschicht weiterhin eine darauf angeordnete hydrophile Beschichtung umfasst.

2. Artikel nach Anspruch 1, wobei die Übertragungsschicht eine gewebte Polyolefinschicht oder eine Polyolefinvliesschicht umfasst.

3. Artikel nach Anspruch 1 oder 2, wobei die Absorptionsschicht eine luftgelegte Schicht umfasst, die gegebenenfalls natürlichen Flockenzellstoff und Bindefasern umfasst.

4. Artikel nach Anspruch 3, wobei die Dichte der luftgelegten Schicht 200 Gramm pro Quadratmeter (GSM) bis 750 GSM, gegebenenfalls 200 GSM bis 350 GSM, beträgt.

5. Artikel nach einem der Ansprüche 1-4, wobei das eine oder die mehreren Indikatorzusammensetzungspolymere Celluloseacetat und/oder Celluloseacetatbutyrat umfassen.

6. Artikel nach einem der Ansprüche 1-5, wobei der nichtcyclische Weichmacher Triethylcitrat ist.

7. Artikel nach einem der Ansprüche 1-6, wobei das hydrophile Tensid Poloxamer 407 ist.

8. Artiekl nach einem der Ansprüche 1-7, wobei die aliphatische C₁₋₁₆-Carbonsäure Citronensäure ist.

9. Artikel nach einem der Ansprüche 1-8, wobei das Ionenausgleichsreagens Tridodecylmethylammoniumchlorid ist.

10. Artikel nach einem der Ansprüche 1-9, wobei das Indikatormittel aus Nitrazingelb, Kresolrot, Alizarin, Bromkresolpurpur, Chlorphenolrot, Bromthymolblau, Bromxylenolblau, Neutralrot, Phenolrot, Thymolblau, Xylenolblau oder m-Kresollila ausgewählt ist.

11. Artikel nach einem der Ansprüche 1-10, wobei die Indikatorzusammensetzung 43,8 Gew.-% Celluloseacetat, 17,5 Gew.-% Celluloseacetatbutyrat, 25,0 Gew.-% Triethylcitrat, 6,6 Gew.-% Poloxamer 407, 5,5 Gew.-% Citronensäure, 1,2 Gew.-% Tridodecylmethylammoniumchlorid und 0,53 Gew.-% Nitrazingelb umfasst.

12. Artikel nach einem der Ansprüche 1-12, wobei die Indikatorzusammensetzung aus Celluloseacetat, Celluloseacetatbutyrat, Triethylcitrat, Poloxamer 407, Citronensäure, Tridodecylmethylammoniumchlorid und Nitrazingelb besteht.

13. Artikel nach Anspruch 1, wobei:
die Membranschicht eine Polypropylenvliesschicht umfasst;
die Übertragungsschicht eine Polypropylenvliesschicht umfasst; und
die absorbierende Schicht eine luftgelegte Schicht umfasst, die ein superabsorbierendes Polymer umfasst, wobei die Dichte der luftgelegten Schicht 200 GSM bis 350 GSM beträgt,
und wobei weiterhin:
die Indikatorzusammensetzung 39 - 47 Gew.-% Celluloseacetat, 15 - 20 Gew.-% Celluloseacetatbutyrat, 22 - 28 Gew.-% Triethylcitrat, 5 - 8 Gew.-% Poloxamer 407, 5 - 8 Gew.-% Citronensäure, 1,15 - 1,27 Gew.-% Tridodecylmethylammoniumchlorid und 0,48 - 0,6 Gew.-% Nitrazingelb umfasst.

## Revendications

1. Article pour la détection de liquide amniotique, comprenant :
une couche de membrane (1) ;
une couche de transfert (2) ayant un premier côté et un second côté ;
une couche absorbante (3) ayant un premier côté et un second côté ; et
une couche de base (4), dans lequel :
la couche de membrane est adjacente au premier côté de la couche de transfert ;
le second côté de la couche de transfert est adjacent au premier côté de la couche absorbante ; et
le second côté de la couche absorbante est adjacent à la couche de base,
et en outre dans lequel :
la couche de transfert comprend une composition d'indicateur disposée sur celle-ci, comprenant :
un ou plusieurs polymères de composition d'indicateur ;
un plastifiant non cyclique ;
un tensioactif hydrophile ;
un acide carboxylique aliphatique en C₁₋₁₆ ;
un réactif d'équilibre ionique ; et
un agent indicateur ;
dans lequel la couche de membrane comprend une couche de polyoléfine tissée ou une couche de polyoléfine non tissée, et
**caractérisé en ce que**
le plastifiant non cyclique est un acide carboxylique aliphatique en C₁₋₁₆ choisi parmi l'acide citrique, l'acide oxalique, l'acide tartrique, l'acide succinique, l'acide glutarique, l'acide lactique, l'acide pyruvique, l'acide hydroxypropionique, l'acide hydroxyvalérique, l'acide adipique et l'acide subérique, ou une combinaison de ceux-ci ; et **en ce que**
la couche de membrane comprend en outre un revêtement hydrophile disposé sur celle-ci.

2. Article selon la revendication 1, dans lequel la couche de transfert comprend une couche de polyoléfine tissée ou une couche de polyoléfine non tissée.

3. Article selon les revendications 1 et 2, dans lequel la couche absorbante comprend une couche appliquée par voie aérienne qui comprend éventuellement de la pulpe de duvet naturelle et des fibres de liaison.

4. Article selon la revendication 3, dans lequel la densité de la couche appliquée par voie aérienne est de 200 grammes par mètre carré (GSM) à 750 GSM, éventuellement de 200 GSM à 350 GSM.

5. Article selon l'une quelconque des revendications 1 à 4, dans lequel le ou les polymères de composition d'indicateur comprennent de l'acétate de cellulose et/ou de l'acétate butyrate de cellulose.

6. Article selon l'une quelconque des revendications 1 à 5, dans lequel le plastifiant non cyclique est le citrate de triéthyle.

7. Article selon l'une quelconque des revendications 1 à 6, dans lequel le tensioactif hydrophile est le poloxamère 407.

8. Article selon l'une quelconque des revendications 1 à 7, dans lequel l'acide carboxylique aliphatique en C₁₋₁₆ est l'acide citrique.

9. Article selon l'une quelconque des revendications 1 à 8, dans lequel le réactif d'équilibre ionique est le chlorure de tridodécylméthylammonium.

10. Article selon l'une quelconque des revendications 1 à 9, dans lequel l'agent indicateur est choisi parmi le jaune de nitrazine, le rouge de crésol, l'alizarine, le pourpre de bromocrésol, le rouge de chlorophénol, le bleu de bromothymol, le bleu de bromoxylénol, le rouge neutre, le rouge de phénol, le bleu de thymol, le bleu de xylénol ou le pourpre de m-crésol.

11. Article selon l'une quelconque des revendications 1 à 10, dans lequel la composition d'indicateur comprend 43,8 % en poids d'acétate de cellulose, 17,5 % en poids d'acétate butyrate de cellulose, 25,0 % en poids de citrate de triéthyle, 6,6 % en poids de poloxamère 407, 5,5 % en poids d'acide citrique, 1,2 % en poids de chlorure de tridodécylméthylammonium et 0,53 % en poids de jaune de nitrazine.

12. Article selon l'une quelconque des revendications 1 à 12, dans lequel la composition d'indicateur est constituée d'acétate de cellulose, d'acétate butyrate de cellulose, de citrate de triéthyle, de poloxamère 407, d'acide citrique, de chlorure de tridodécylméthylammonium et de jaune de nitrazine.

13. Article selon la revendication 1, dans lequel :
la couche de membrane comprend une couche de polypropylène non tissé ;
la couche de transfert comprend une couche de polypropylène non tissé ; et
la couche absorbante comprend une couche appliquée par voie aérienne comprenant un polymère superabsorbant, dans laquelle la densité de la couche appliquée par voie aérienne est de 200 GSM à 350 GSM,
et en outre dans lequel :
la composition d'indicateur comprend 39 à 47 % en poids d'acétate de cellulose, 15 à 20 % en poids d'acétate butyrate de cellulose, 22 à 28 % en poids de citrate de triéthyle, 5 à 8 % en poids de poloxamère 407, 5 à 8 % en poids d'acide citrique, 1,15 à 1,27 % en poids de chlorure de tridodécylméthylammonium et 0,48 à 0,6 % en poids de jaune de nitrazine.
